# EUROPEAN PATENT SPECIFICATION

(11) **EP 3 313 364 B1**
(45) Date of publication and mention of the grant of the patent: **23.10.2019**
(21) Application number: 15731061.6
(22) Date of filing: 24.06.2015
(51) Int. Cl.: A61K 8/81, A61Q 5/04, A61Q 5/06, A61Q 5/00

(54) **HAIR TREATMENT PROCESS**
VERFAHREN ZUR HAARBEHANDLUNG
PROCEDE DE TRAITEMENT DES FIBRES KERATINIQUES

(43) Date of publication of application: 02.05.2018
(73) Proprietor: Kao Germany GmbH, 64297 Darmstadt (DE)
(72) Inventor: HOFFMANN, Martin, 64297 Darmstadt Hessen (DE); NING, Jing, 64297 Darmstadt Hessen (DE)
(86) International application number: PCT/EP2015/064285
(87) International publication number: WO 2016/206738

(56) References cited:
- EP-A1- 2 011 478
- EP-A1- 2 191 864
- WO-A1-2009/102758
- WO-A2-2013/087316

## Description

The present invention relates to a hair treatment process wherein an aqueous composition comprising one or more cationic quaternary ammonium polymers having a cationic charge density of 3.0 mEq/g or more and having a pH in the range of 3.0 to 6.5 is applied onto hair, optionally left on the hair for a period of 1 to 15 min, optionally rinsed off from hair and the hair temperature is increased to the range of 80 to 140°C. The novel treatment process allows consumers to a long lasting conditioning of their hair.

Hair conditioners are widely used for improving hair combability and suppleness among other properties which are as well improved and/or given. Consumers use the conditioners after each shampooing as hair combability and suppleness is usually reduced after cleansing the hair surface. Such process takes long time under shower and therefore there is a great need for hair conditioners which provides hair long lasting combability and suppleness improvement. In other words, the consumers do not need to use a conditioning composition after each hair shampooing for improving combability and suppleness for certain number of hair washes.

The above mentioned problems have been subject of numerous research projects. The one approach has been improving hair conditioning properties of cleansing compositions so that there is no need for the use of an additional composition just for making hair better combable and supple. In this way there have been many product initiatives on the market in form of so called two-in-one shampoo compositions.

DE 10 2013225898 discloses compositions for long lasting hair conditioning comprising cationic polymer with a relatively high charge density. It has been observed that compositions disclosed do not really provide the long lasting conditioning effects because of several reasons which is made clear below.

The inventors of the present invention have surprisingly found when an aqueous composition comprising cationic quaternary ammonium polymer having certain cationic charge density is applied onto hair and optionally after leaving on the hair for a defined period of time, and optionally rinsing off the composition form hair, the hair temperature is increased significantly above the ambient temperature, the conditioning effect provided to the hair especially in terms of combability and suppleness is held with repeated hair washes up to 20 wash cycles.

Accordingly, the first object of the present invention is a process for treating hair wherein an aqueous composition comprising one or more cationic quaternary ammonium polymers having a cationic charge density of 3.0 mEq/g or more is applied onto hair, optionally left on the hair for a period of 1 to 15 min, optionally rinsed off from hair and the hair temperature is increased to the range of 40 to 140°C.

Second object of the present invention is the use of the process of the present invention for long lasting conditioning of human hair, especially improving combability and suppleness of hair.

The third object of the present invention is a kit for treating human hair comprising the aqueous composition used in the process of the present invention and optionally a device which makes the increase of hair temperature possible.

The aqueous composition used in the process of the present invention comprises one or more cationic quaternary ammonium polymers having a charge density of 3 mEq/g or more, preferably 3.5 to 8 mEq/g, more preferably 4 to 7.5 mEq/g, most preferably 4.5 to 7.0 mEq/g and in particular 4.5 to 6.5 mEq/g. In a preferred embodiment of the present invention the aqueous composition comprises only one cationic quaternary ammonium polymer with the above defined cationic charge density ranges.

Suitable non-limiting cationic quaternary ammonium polymers for the purpose of the present invention are Polyquaternium-37, Polyquaternium-6, Polyquaternium-7 and Polyquaternium-16.

The preferred cationic polymers are Polyquaternium-37 and Polyquaternium-16. The particularly preferred cationic polymer is Polyquaternium-37

Total cationic quaternary ammonium polymer concentration having the above described cationic charge density is in the range of 0.1 to 5%, preferably 0.2 to 4%, more preferably 0.25 to 3% and most preferably 0.3 to 2.5% by weight, calculated to the total composition.

According to the process of the present invention the hair is heated up to a temperature in the range of 80 to 140 °C. The heating of the hair is achieved with a hair dryer, with digital hair heating devices, with an iron which may be either flat or having certain surface structures or a round shaped iron. The operation of such kind of devices is commonly known by the skilled in the art or by the users who may be seen as average users of such devices.

In a preferred embodiment of the process the aqueous composition is not rinsed off from hair prior to increasing the temperature of the hair.

The aqueous composition used in the process of the present invention comprises preferably one or more nonionic surfactants. Suitable ones are fatty alcohol ethoxylates of the following general structure

R₁ (OCH₂CH₂)ₙ OH

wherein R₁ is straight or branched, saturated or unsaturated alkyl chain which may be synthetic or natural with a C chain length in the range of 8 to 40, preferably 9 to 30 and more preferably 9 to 24 and n is a number in the range of 5 to 40, preferably 9 to 30.

Non-limiting suitable examples of the fatty alcohol ethoxylates are C9-11 Pareth-6, C9-11 Pareth-8, C9-15 Pareth-8, C11-13 Pareth-9, C11-13 Pareth-10, C11-15 Pareth-5, C11-15 Pareth-7, C11-15 Pareth-9, C11-15 Pareth-12, C11-15 Pareth-15, C11-15 Pareth-20, C11-15 Pareth-30, C11-15 Pareth-40, C11-21 Pareth-10, C12-13 Pareth-5, C12-13 Pareth-6, C12-13 Pareth-7, C12-13 Pareth-9, C12-13 Pareth-10, C12-13 Pareth-15, C12-13 Pareth-23, C12-14 Pareth-5, C12-14 Pareth-7, C12-14 Pareth-9, C12-14 Pareth-11, C12-14 Pareth-12, C12-15 Pareth-5, C12-15 Pareth-7, C12-15 Pareth-9, C12-15 Pareth-10, C12-15 Pareth-11, C12-15 Pareth-12, C12-16 Pareth-5, C12-16 Pareth-7, C12-16 Pareth-9, C13-15 Pareth-21, C14-15 Pareth-7, C14-15 Pareth-8, C14-15 Pareth-11, C14-15 Pareth-12, C14-15 Pareth-13, C20-22 Pareth-30, C20-40 Pareth-10, C20-40 Pareth-24, C20-40 Pareth-40, C20-40 Pareth-95, C22-24 Pareth-33, Beheneth-5, Beheneth-10, Beheneth-15, Beheneth-20, Beheneth-25, Beheneth-30, Ceteareth-5, Ceteareth-6, Ceteareth-7, Ceteareth-10, Ceteareth-11, Ceteareth-12, Ceteareth-15, Ceteareth-20, Ceteareth-25, Ceteareth-30, Ceteareth-35, Ceteareth-40, Laureth-5, Laureth-10, Laureth-15, Laureth-20, Laureth-25, Laureth-30, Laureth-40, Myreth-5, Myreth-10, Ceteth-5, Ceteth-10, Ceteth-15, Ceteth-20, Ceteth-25, Ceteth-30, Ceteth-40, Oleth-5, Oleth-10, Oleth-15, Oleth-20, Oleth-25, Oleth-30, Oleth-40, Steareth-5, Steareth-10, Steareth-15, Steareth-20, Steareth-25, Steareth-30, Steareth-35, and Steareth-40. They may also be comprised in the compositions as a mixture of more than one surfactant.

Further suitable nonionic surfactant is polypropylene glycol ether of fatty alcohols according to general structure

R₂(OCH₂(CH₃)CH₂)ₙ OH

wherein R₂ is straight or branched, saturated or unsaturated fatty alcohol which may be synthetic or natural with a C chain length in the range of 8 to 40, preferably 9 to 30 and more preferably 9 to 24 and n is a number in the range of 1 to 40, preferably 3 to 30.

Suitable non-limiting examples are PPG-3 Caprylyl ether, PPG-5 Caprylyl ether, PPG-10 Caprylyl ether, PPG-10 Cetyl ether, PPG-20 Cetyl ether, PPG-28 Cetyl ether, PPG-30 Cetyl ether, PPG-7 Lauryl ether, PPG-10 Lauryl ether, PPG-10 Oleyl ether, PPG-20 Oleyl ether, PPG-23 Oleyl ether, PPG-30 Oleyl ether, PPG-11 Stearyl ether and PPG-15 Stearyl ether.

Further suitable nonionic surfactants are polyethylene glycol fatty acid esters of the following general structure

R₃ C(O) (OCH₂CH₂)ₙ OH

wherein R₃ is straight or branched, saturated or unsaturated alkyl group which may be synthetic or natural with a C chain length in the range of 7 to 39, preferably 9 to 29 and more preferably 9 to 23 and n is a number in the range of 5 to 40, preferably 9 to 30.

Suitable non-limiting examples are PEG-8 Behenate, PEG-8 Caprate, PEG-8 Caprylate, PEG-5 Cocoate, PEG-8 Cocoate, PEG-9 Cocoate, PEG-10 Cocoate, PEG-15 Cocoate, PEG-6 Isopalmitate, PEG-6 Isostearate, PEG-8 Isostearate, PEG-9 Isostearate, PEG-10 Isostearate, PEG-12 Isostearate, PEG-20 Isostearate, PEG-30 Isostearate, PEG-40 Isostearate, PEG-6 Laurate, PEG-8 Laurate, PEG-9 Laurate, PEG-10 Laurate, PEG-12 Laurate, PEG-14 Laurate, PEG-20 Laurate, PEG-30 Laurate, PEG-8 Myristate, PEG-20 Myristate, PEG-5 Oleate, PEG-6 Oleate, PEG-7 Oleate, PEG-8 Oleate, PEG-9 Oleate, PEG-10 Oleate, PEG-11 Oleate, PEG-12 Oleate, PEG-15 Oleate, PEG-20 Oleate, PEG-30 Oleate, PEG-32 Oleate, PEG-6 Palmitate, PEG-18 Palmitate, PEG-20 Palmitate, PEG-5 Stearate, PEG-6 Stearate, PEG-7 Stearate, PEG-8 Stearate, PEG-9 Stearate, PEG-10 Stearate, PEG-12 Stearate, PEG-14 Stearate, PEG-15 Stearate, PEG-20 Stearate, PEG-25 Stearate, PEG-30 Stearate, PEG-35 Stearate and PEG-40 Stearate.

Further suitable nonionic surfactants are polypropylene glycol fatty acid esters of the following general structure

R₄ C(O) (OCH₂(CH₃)CH₂)ₙ OH

wherein R₄ is straight or branched, saturated or unsaturated alkyl group which may be synthetic or natural with a C chain length in the range of 7 to 39, preferably 9 to 29 and more preferably 9 to 23 and n is a number in the range of 1 to 40, preferably 9 to 30.

Suitable non-limiting examples are PPG-15 Isostearate, PPG-9 Laurate, PPG-26 Oleate and PPG-36 Oleate.

Further nonionic suitable surfactants are polyethylene glycol and polypropylene glycol ether of fatty alcohols of the following general structure

R₅ (OCH₂(CH₃)CH₂)ₙ₁ (OCH₂CH₂)ₙ₂ OH

wherein R₅ is straight or branched, saturated or unsaturated alkyl group which may be synthetic or natural with a C chain length in the range of 7 to 39, preferably 9 to 29 and more preferably 9 to 23 and n1 and n2 may be the same or different and are a number in the range of 1 to 40.

Suitable non-limiting examples are PPG-2 Ceteareth-9, PPG-4 Ceteareth-12, PPG-4 Ceteareth-20, PPG-2 C9 - 11 Pareth-5, PPG-2 C9 - 11 Pareth-7, PPG-2 C9 - 11 Pareth-8, PPG-2 C9 - 11 Pareth-11, PPG-2 C12 - 13 Pareth-8, PPG-2 C12 - 15 Pareth-6, PPG-4 C 13- 15 Pareth-15, PPG-5 C9 - 15 Pareth-6, PPG-6 C9 - 11 Pareth-5, PPG-6 C12 - 15 Pareth-12, PPG-6 C12 - 18 Pareth-11, PPG-1 Deceth-4, PPG-1 Deceth-5, PPG-1 Deceth-6, PPG-1 Deceth-7, PPG-2 Deceth-3, PPG-2 Deceth-7, PPG-2 Deceth-8, PPG-2 Deceth-10, PPG-2 Deceth-15, PPG-2 Deceth-20, PPG-2 Deceth-30, PPG-2 Deceth-40, PPG-4 Deceth-4, PPG-4 Deceth-6, PPG-4 Deceth-6, PPG-6 Deceth-4, PPG-6 Deceth-9, PPG-8 Deceth-6, PPG-14 Deceth-6, PPG-2 Laureth-5, PPG-2 Laureth-8, PPG-2 Laureth-12, PPG-3 Laureth-8, PPG-3 Laureth-9, PPG-3 Laureth-10, PPG-3 Laureth-12, PPG-4 Laureth-2, PPG-4 Laureth-5, PPG-4 Laureth-7, PPG-4 Laurreth-15, PPG-5 Laureth-5, PPG-5 Laureth-3, PPG-20 Laureth-12, PPG-25 Laureth-25, PPG-3 Myreth-3, PPG-3 Myreth-11, PPG-9 Steareth-3, PPG-23 Steareth-34, PPG-30 Steareth-4, PPG-34 Steareth-3, and PPG-38 Steareth-6.

The aqueous composition may further suitably comprise ethoxylated triglycerides as nonionic surfactants. Well known and commonly used ones are ethoxylated castor oil such as PEG-40 hydrogenated castor oil or and PEG-60 hydrogenated castor oil.

Total concentration of non-ionic surfactants is in the range of 0.1 to 5%, preferably 0.2 to 4%, more preferably 0.25 to 3% and most preferably 0.3 to 2.5% by weight, calculated to the total composition.

The aqueous composition used in the process of the present invention preferably comprises one or more aminated silicones. The suitable aminated silicones are the ones with primary or secondary or tertiary amine group bearing silicones. The quaternary ammonium group bearing silicones are not suitable as soon as their concentration exceeds 10%, preferably 5% of the total concentration of the one or more cationic polymer having charge density as defined above in the composition.

Suitable aminated silicones are according to the general structure 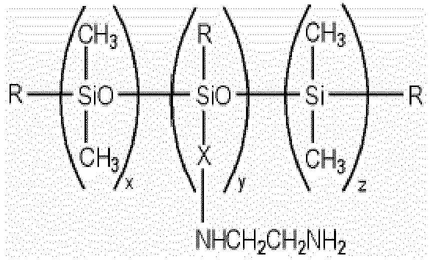 wherein R is the same or different OH or CH₃ or OCH₃ and X represents butyl, propyl, isopropyl or isobutyl.

The especially preferred aminated silicone is Bis(Hydroxy/Methoxy) Amodimethicone wherein X is isobutyl and one of the R is OH and the other is OCH₃ in the above general structure.

Total concentration of one or more aminated silicones is in the range of 0.1 to 5%, preferably 0.2 to 4%, more preferably 0.25 to 3% and most preferably 0.3 to 2.5% by weight, calculated to the total composition.

In a preferred embodiment of the present invention the aqueous composition used in the process of the present invention does not comprise quaternary ammonium surfactants at high concentrations. It has been observed that when quaternary ammonium surfactants are comprised in the composition at a concentration above 10% preferably 5% of the total concentration of the one or more cationic polymers as defined above, the long lasting effect of the aqueous composition is dramatically diminished. Without being bound by the theory, one explanation might be that the amount of adsorbed / bound cationic polymer concentration is decreased by co-adsorption of the cationic surfactants. Accordingly, the aqueous composition of the present invention does not comprise quaternary ammonium surfactants at a concentration of 10%, preferably 5% or more, by weight, of the total concentration of cationic polymer as defined above.

Specifically, the aqueous composition of the present invention does not comprise quaternary ammonium surfactant of the general structure below at a concentration more than 10%, preferably 5% by weight of the total concentration of one or more cationic quaternary ammonium polymers having the cationic charge density as defined above. The general structure of cationic quaternary ammonium surfactant is

R₁₁ R₁₂ R₁₃ R₁₄ N

wherein R₁₁ is an alky chain having C length of 8 to 30 which may be saturated or unsaturated, straight or branched, R₁₂ an alky chain having C length of 1 to 30 which may be saturated or unsaturated, straight or branched, R₁₁ and R₁₂ additionally may take the structures of

R₁₅ C(O) O (CH2) n or R₁₅ C(O) NH (CH2)n

wherein R15 is an alkyl chain with a C length of 7 to 29 which may be saturated or unsaturated, straight or branched and n is a number between 1 and 4, R₁₃ and R₁₄ is an alkyl chain with a C length of 1 to 4 which may be straight or branched (only for C3 and C4), wherein all alkyl chains may include one or more substituents such as hydroxyl or (poly)ethoxy groups.

The composition used in the process of the present invention may comprise one or more fatty alcohols and may be in a form of emulsion. Suitable non-limiting examples are according to general structure

R₆-OH

wherein R₆ is straight or branched, saturated or unsaturated alkyl chain which may be synthetic or natural with a C chain length in the range of 12 to 24 which may as well be substituted with one or more groups on the alkyl chain.

Non limiting suitable examples are lauryl alcohol, myristyl alcohol, cetyl alcohol, oleyl alcohol, stearyl alcohol, behenyl alcohol and their mixtures. As a mixed fatty alcohol the mostly used one is the cetearyl alcohol as well preferred in the compositions of the present invention.

The total concentration of one or more fatty alcohols is in the range of 0.1 to 20%, preferably 0.5 to 15%, more preferably 1 to 10% and the most preferably 2 to 7.5% by weight calculated to the total composition.

The aqueous composition used in the process of the present invention may comprise one or more organic solvents. Suitable ones are ethanol, propanol, isopropanol, benzyl alcohol, benzyloxyethanol, ethoxydiglycol, alkylene carbonates such as ethylene carbonate and propylene carbonate, phenoxyethanol, butanol, isobutanol, cyclohexane, cyclohexanol, hexyleneglycol, ethylenecarbonate, propyleneglycol, poypropyleneglycols, ethyleneglycol monoethylether, ethylene glycol monobutyl ether, ethylene glycol monophenyl ether, 1-phenylethylalcohol, 2-phenylethylalcohol, o-methoxyphenol. The most preferred ones are ethanol, isopropanol, benzyl alcohol and polypropylene glycols. Concentration of organic solvents should not exceed 20% by weight, preferably in the range of 0.1 to 15 %, more preferably 0.1 to 10% by weight and most preferably 0.1 to 7.5% by weight calculated to total composition. It should be noted that concentration of one or more organic solvents is very much dependent on the type of preparation i.e. a solution can contain higher concentration of organic solvent than a gel or emulsion composition.

Further in an embodiment of the present invention, the aqueous composition used in the process of the present invention comprises at least one hair direct dye selected from cationic and neutral nitro dyes.

Any cationic direct dye is in principal suitable for the compositions. Non-limiting suitable examples are Basic Blue 6, Basic Blue 7, Basic Blue 9, Basic Blue 26, Basic Blue 41, Basic Blue 99, Basic Blue 124, Basic Brown 4, Basic Brown 7, Basic Brown 16, Basic Brown 17, Basic Green 1, Basic Orange 31, Basic Red 2, Basic Red 12, Basic Red 22, Basic Red 51, Basic Red 76, Basic Violet 2, Basic Violet 3, Basic Violet 10, Basic Violet 14, Basic Violet 57 and Basic Yellow 87.

Neutral dyes, so called nitro dyes for shading purposes are also optionally contained in the compositions. Suitable ones are HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No.12, HC Yellow No.13, HC Yellow No.14, HC Yellow No.15, 2- Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid.

Concentration of one or more direct dyes in total is in the range of 0.001 to 1.0% by weight, preferably 0.01 to 0.8% more preferably 0.05 to 0.75% and most preferably 0.1 to 0.5% by weight calculated to total composition.

The aqueous composition used in the process of the present invention may further comprise one or more UV filters which may be selected from water soluble ones as well as oils soluble ones. The oil soluble UV filter are more preferred ones as they show no interaction with the cationic quaternary ammonium polymers. Non-limiting examples are 4-Aminobenzoic acid and the esters and salts thereof, 2-phenyl benzimidazole-5-sulfonic acid and the alkali and amine salts thereof, 4-dimethyl aminobenzoic acid and the esters and salts thereof, cinnamic acid and the esters and salts thereof, 4-methoxycinnamic acid and the esters and salts thereof, salicylic acid and the esters and salts thereof, 2.4-dihydroxybenzophenone, 2.2'.4.4'-tetrahydroxybenzophenone, 2-hydroxy-4-methoxybenzophenone and its 5-sulfonic acid or the sodium salt thereof, 2.2'-dihydroxy-4.4'-dimethoxybenzophenone, 2-hydroxy-5-chlorobenzophenone, 2.2'-dihydroxy-4-methoxybenzophenone, 2.2'-dihydroxy-4.4'-dimethoxy-5.5'-disulfobenzo-phenone or the sodium salt thereof, 2-hydroxy-4-octyloxybenzophenone, 2-hydroxy-4-methoxy-4'-methylbenzophenone, 3-benzyl-idenecampher, 3-(4'-sulfo)-benzyl-idenebornane-2-one and the salts thereof, 3-(4'-methyl benzylidene)-DL-campher, and/or polysilicone-15.

The total UV filter concentration may be in the range of 0.01 to 1 % by weight calculated to the total composition.

The composition used in the process of the present invention may comprise oil and/or oily substances which are suitably selected from silicone oils, either volatile or non-volatile, natural and synthetic oils, fatty alcohol ethers (dialkyl ethers) and fatty acid fatty alcohol esters. Among silicone oils those can be added to the compositions include, dimethicone, dimethiconol, polydimethylsiloxane (DC fluid ranges from Dow Corning), arylated silicones such as phenyl methicone, phenyl trimethicone, diphenyl dimethicone, diphenylsiloxy phenyl trimethicone, tetramethyl tetraphenyl trisiloxane, triphenyl trimethicone, and trimethyl pentaphenyl trisiloxane, aqueous emulsion of divinyldimethicone/dimethicone copolymer, preferably with a viscosity of higher than 1 x 10⁸ mm²/s, more preferably higher than 1.1 x 10⁸ mm²/s measured at 0.01 Hz and at approximately 25°C.

Suitable non-limiting examples to natural plant oils are such as olive oil, almond oil, avocado oil, wheatgerm oil and ricinus oil.

Suitable non-limiting examples to fatty alcohol fatty acid esters are isopropyl myristate, palmitate, stearate and isostearate, oleyl oleate, isocetyl stearate, hexyl laurate, dibutyl adipate, dioctyl adipate, myristyl myristate and oleyl erucate.

Suitable non-limiting examples to fatty alchol ethers (dialkyl ethers) are such as dimyristyl ether, dicetyl ether and dicaprylyl ether.

The compositions comprise oil and/or oily substances at a total concentration in the range of 0.01 to 10%, preferably 0.05 to 7.5%, more preferably 0.1 to 5 and most preferably 0.1 ta 3% by weight calculated to total composition.

The compositions according to the invention may also comprise further conditioning substances such as protein hydrolyzates and polypeptides, e.g., keratin hydrolyzates, collagen hydrolyzates of the type "Nutrilan^{R}" or elastin hydrolyzates, as well as also in particular plant protein hydrolyzates, optionally, cationized protein hydrolyzates, e.g., "Gluadin^{R}".

The composition used in the process of the present invention of the present invention may further comprise natural plant extracts. Within the meaning of the present invention the extracts are liquid extracts and prepared by mixing plant parts such as leaves, fruits, blossoms and roots with a solvent such as water, alcohol, propyleneglycol or mixture of more than one solvent and incubating for certain period of time and filtrating the undissolved plant parts. Suitable aqueous (e.g. steam-distilled) alcoholic or hydro-alcoholic plant extracts known **per se** are in particular aloe, pineapple, artichoke, arnica, avocado, valerian, bamboo, henbane, birch, stinging nettle, echinacea, ivy, wild angelica, gentian, ferns, pine needles, silver weed, ginseng, broom, oat, rose hip, hamamelis, hay flowers, elderberry, hop, coltsfoot, currants, chamomile, carrots, chestnuts, clover, burr root, cocoanut, cornflower, lime blossom, lily of the valley, marine algae, balm, mistletoe, passion flower, ratanhia, marigold, rosemary, horse chestnut, pink hawthorn, sage, horsetail, yarrow, primrose, nettle, thyme, walnut, wine leaves, white hawthorn, etc. Suitable trade products are, for example, the various "Extrapon®" products, "Herbasol^{R}", "Sedaplant^{R}" and "Hexaplant^{R}". Extracts and the preparation thereof are also described in "Hagers Handbuch der pharmazeutischen Praxis", 4th Ed. Preferred plant extracts are prepared from Vitis vinifera, Malus domestica, Camelia sinensis, Juglans regia Ribes Uva-Crispa, Ribes nigrum, Ribes rubrum and Punica granatum. The above mentioned extracts may also be available in the powder form and such are also suitable within the meaning of the present invention.

The natural plant extracts are included into the compositions at a concentration of 0.001 to 1.0%, preferably 0.005 to 0.75%, more preferably 0.01 to 0.6% and most preferably 0.05 to 0.5% by weight, calculated to total composition based on dry matter of the extract.

Further in an embodiment of the present invention, compositions comprise one or more ubiquinone of the following general structure 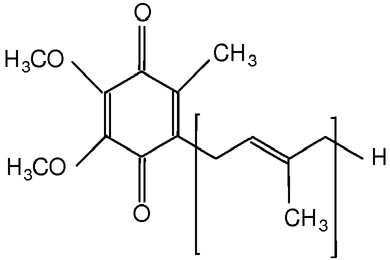 where n is a number between 1 and 10. It should be noted that the compositions of the present invention can certainly comprise more than one ubiquinone. Preferred ubiquinones are the ones where n is a number between 6 and 10 and especially preferred is Ubiquinone 50 where n is 10, also known as Coenzyme Q10. Concentration ubiquinone of the above formula in the compositions is from 0.0001 to 1%, preferably from 0.0002 to 0.75%, more preferably from 0.0002 to 0.5% and most preferably from 0.0005 to 0.5% by weight, calculated to total composition.

The pH of the compositions according to the present invention is suitably between 3.0 and 6.5, more preferably 4.0 to 5.5 and most preferably 4.0 to 5.

In principal pH of the compositions can be adjusted with any organic and/or inorganic acids or their mixture. Some of them to mention are phosphoric acid, hydrochloric acid as the inorganic ones and to the organic acids the well-known citric acid and lactic acid, glycolic acid, glyoxylic acid, hydroxyacrylic acid, glyceric acid, malic acid and tartaric acid and of the dicarboxylic acids are malonic acid, succinic acid, glutaric acid, adipic acid, maleic acid, fumaric acid and phtalic acid.

Conditioning compositions of the present invention can comprise additionally any compound customarily found in conditioning compositions such as chelating agents, preservatives and fragrance.

The sequestering agents are selected from polycarboxy acids. The preferred one is ethylene diamine tetraacetic acid, EDTA. Typical useful concentration range for sequestering agents is of 0.01 - 2.5% by weight calculated to the total composition.

Viscosity of the conditioning composition may be adjusted according to the application form and generally should not be more than 50,000 mPa.s at 20°C measured with Brookfield Rheometer at a shear rate of 5 sec⁻¹.

Thickening agents especially the nonionic thickening agents may be added into the compositions of the present invention. Suitable non-limiting examples are cellulose derivatives such as, methyl cellulose, hydroxyethyl cellulose, hydroxymethyl cellulose, hydroxypropyl cellulose, guar gum and its derivatives, and cognac mannan and derivatives. Thickeners may be included at a concentration of 0.05 to 2.5% by weight calculated to total composition. Concentration of thickener is very much dependent on the thickener itself and also the preparation such as pH value of the composition etc. and therefore should be selected depending on the desired viscosity of the composition.

The aqueous composition of the present invention may be in the form of solution, gel, dispersion and emulsion. These preparations may be provided to the consumers as they are confectioned in a suitable packaging which allows easy release of the products and distribution onto hair as well as they may be provided in a pressurized container. In case pressurized container is used as the vessel carrying the composition, it is additionally comprised a pressurizing gas which may be selected form the any known ones such as alkanes , butane, isobutene, dimethyl ether, pressurized air, nitrogen, carbon dioxide etc.

The following examples are to illustrate the invention but not to limit it.

### Example 1

The following compositions were prepared for the comparative tests.

| | % active matter | | |
|---|---|---|---|
| | A | B | C |
| Polyquaternium 37 | 0.8 | 0.8 | 0.8 |
| C12-13 Pareth-9 | - | 0.5 | 0.5 |
| PPG-3 Caprylyl ether | - | - | 1.0 |
| Bis (Hydroxy/Methoxy) Amodimethicone | - | - | 0.5 |
| Citric acid | q.s. to pH 4.0 | | |
| Water | to 100 | | |

All three compositions are within the scope of the claim 1.

Human hair streaks having approximately 25 cm length and weighing 3.5 g was used for the comparative tests. The streak was damaged prior to the tests by means of double bleaching with a bleaching composition commercially available under the brand name Goldwell.

The streaks, always in duplicate, were treated with the above compositions. Therefore, each streak was applied approximately 1 g of the product and left on the streak for 15 min and the streaks were rinsed off with water. Afterwards only one of the streaks of each pair was dried with a hair drier. Hair temperature reached was approximately 95°C. The temperature measurement was carried out using an infrared measuring device from a distance of approximately 5 cm.

Additionally two hair streaks were treated with composition C and the one was heated using hair drier to a temperature of 95°C without rinsing off the composition (C+* in the Table below) and the other was treated with an hot iron operated at 130°C for 5 times which resulted in approximate hair temperature of 100°C (C+** in the Table below).

The suppleness of the hair was measured with a zwick machine and expressed as the force (in milliNewton - mN) to pull off a streak through the rods of the machine. The machine is commercially available from Zwick/Roell Company and includes a manual. It was used according to its manual. Briefly, the streak was places between the metal rods placed offset in a given distance horizontally (2 cm) and vertically (8 cm) 5 in total and the force needed to pull the streaks through the metal rods at a given rate (5 cm/min) was measured. All values reported in the Table below are average of at least 6 readings.

In order to test the long lastingness of hair suppleness, the hair streaks were washed 5 times first and the suppleness was measured again and after additional wash the measurements were repeated. For washing hair streaks, 5% by weight Sodium lauryl ether sulfate solution in water at pH 5.0 was used.

The following results were obtained.

| | | Suppleness | | |
|---|---|---|---|---|
| | | (mN) | | |
| Composition | Heat | Start | 5 Wash | 10 Wash |
| Water | - | 1741 | 2072 | 1938 |
| A | + | 756 | 873 | 1023 |
| A | - | 791 | 1145 | 1578 |
| B | + | 697 | 809 | 798 |
| B | - | 715 | 1046 | 1311 |
| C | + | 450 | 515 | 659 |
| C | - | 525 | 1020 | 1280 |
| C | +* | 348 | 518 | 629 |
| C | +** | 388 | 489 | 572 |

It should be noted that the lower the value, the higher the suppleness of the hair.

From the above results it is clear that increasing hair temperature does really have a positive effect on the improvement of suppleness of hair right after treatment. It was observed that the suppleness was affected by the presence of additional substances but all the streaks were judged to have enough suppleness.

However, increasing hair temperature has also dramatically affected the suppleness of hair after hair wash cycles. The streaks did not have any temperature increase lost suppleness after 5 washes whereas the streaks treated with heat still had significantly lower suppleness force after 10 hair washes.

The above results prove beyond any doubt the improved suppleness of the hair treated according to the process of the present invention.

The following examples fall within the scope of the present invention.

### Example 2

| | % by weight |
|---|---|
| Polyquaternium-37 | 0.80 |
| C12-13 Pareth-9 | 0.50 |
| Hydroxyethylcellulose | 0.50 |
| Lactic acid | q.s. to pH 4.2 |
| Water | to 100 |

### Example 3

| | % by weight |
|---|---|
| Polyquaternium-37 | 0.80 |
| C12-13 Pareth-9 | 0.50 |
| Hydroxypropyl guar | 0.50 |
| Lactic acid | q.s. to pH 4.2 |
| Water | to 100 |

### Example 4

| | % by weight |
|---|---|
| Polyquaternium-37 | 0.80 |
| C12-13 Pareth-9 | 0.50 |
| Basic Red 51 | 0.20 |
| Basic Orange 37 | 0.02 |
| Basic Yellow 87 | 0.08 |
| Citric acid | q.s. to pH 3.8 |
| Water | to 100 |

### Example 5

| | % by weight |
|---|---|
| Polyquaternium-37 | 0.80 |
| C12-13 Pareth-9 | 0.85 |
| Benzophenone-3 | 0.25 |
| Basic Red 51 | 0.20 |
| Basic Orange 37 | 0.02 |
| Basic Yellow 87 | 0.08 |
| Citric acid | q.s. to pH 3.8 |
| Water | to 100 |

### Example 6

| | % by weight |
|---|---|
| Polyquaternium-37 | 0.80 |
| C12-13 Pareth-9 | 0.85 |
| Benzophenone-3 | 0.25 |
| Ethylhexyl methoxy cinnamate | 0.10 |
| Basic Red 51 | 0.20 |
| Basic Orange 37 | 0.02 |
| HC red no 3 | 0.08 |
| Citric acid | q.s. to pH 3.8 |
| Water | to 100 |

### Example 7

| | % by weight |
|---|---|
| Polyquaternium-37 | 0.80 |
| C12-13 Pareth-9 | 0.85 |
| Benzophenone-3 | 0.25 |
| Ethylhexyl methoxy cinnamate | 0.10 |
| Hydroxypropyl guar gum | 0.25 |
| Basic Red 51 | 0.20 |
| Basic Orange 37 | 0.02 |
| HC red no 3 | 0.08 |
| Citric acid | q.s. to pH 3.8 |
| Water | to 100 |

### Example 8

| | % by weight |
|---|---|
| Polyquaternium-37 | 0.80 |
| Ceteareth 20 | 1.25 |
| Cetearyl alcohol | 5.00 |
| Dimethicone 100 cSt | 0.85 |
| Ethylhexyl methoxy cinnamon | 0.10 |
| Lactic acid | q.s. to pH 3.8 |
| Water | to 100 |

### Example 9

| | % by weight |
|---|---|
| Polyquaternium-37 | 0.80 |
| Ceteareth 30 | 1.25 |
| Cetearyl alcohol | 2.00 |
| Behenyl alcohol | 2.80 |
| Dimethicone 50 cSt | 0.85 |
| Almond oil | 0.10 |
| Vitis vinifera extract | 0.10 (dry matter) |
| Lactic acid | q.s. to pH 4.5 |
| Water | to 100 |

### Example 10

| | % by weight |
|---|---|
| Polyquaternium-37 | 0.80 |
| Ceteareth 30 | 1.25 |
| Cetearyl alcohol | 2.00 |
| Behenyl alcohol | 2.80 |
| Basic Red 51 | 0.20 |
| Basic Orange 37 | 0.02 |
| HC red no 3 | 0.08 |
| Basic yellow 87 | 0.03 |
| HC Yellow 2 | 0.03 |
| Bis(Hydroxy/Methoxy) Amodimethicone | 0.85 |
| Almond oil | 0.10 |
| Vitis vinifera extract | 0.10 (dry matter) |
| Lactic acid | q.s. to pH 4.5 |
| Water | to 100 |

## Claims

1. A process for treating hair wherein an aqueous composition comprising one or more cationic quaternary ammonium polymers having a cationic charge density of 3.0 mEq/g or more and having a pH in the range of 3.0 to 6.5 is applied onto hair, optionally left on the hair for a period of 1 to 15 min, optionally rinsed off from hair and the hair temperature is increased to the range of 80 to 140°C.

2. The process according to claim 1 wherein the hair is heated up with a hair dryer or with a digital hair heating device, with an iron which may be either flat or having certain surface structures or a round shaped iron.

3. The process according to claims 1 or 2 wherein the cationic quaternary ammonium polymer has a charge density of 3.5 to 8 mEq/g, more preferably 4 to 7.5 mEq/g, most preferably 4.5 to 7.0 mEq/g and in particular 4.5 to 6.5 mEq/g.

4. The process according to any one of the claims 1 to 3 wherein the cationic quaternary ammonium polymer is selected from Polyquaternium-37, Polyquaternium-6, Polyquaternium-7, and Polyquaternium-16, preferably it is Polyquaternium-37.

5. The process according to any one of the claims 1 to 5 wherein the aqueous composition comprises one or more cationic quaternary ammonium polymers at a total concentration of 0.1 to 5% by weight calculated to the total composition.

6. The process according to any one of the claims 1 to 6 wherein the aqueous composition comprises one or more nonionic surfactants.

7. The process according to any one of the claims 1 to 7 wherein one or more nonionic surfactants are selected from
a- fatty alcohol ethoxylates of the following general structure
R₁(OCH₂CH₂)ₙ OH
wherein R₁ is straight or branched, saturated or unsaturated alkyl chain which may be synthetic or natural with a C chain length in the range of 8 to 40, preferably 9 to 30 and more preferably 9 to 24 and n is a number in the range of 5 to 40, preferably 9 to 30.
b- polypropylene glycol ether of fatty alcohols according to general structure
R₂(OCH₂(CH₃)CH₂)ₙ OH
wherein R₂ is straight or branched, saturated or unsaturated fatty alcohol which may be synthetic or natural with a C chain length in the range of 8 to 40, preferably 9 to 30 and more preferably 9 to 24 and n is a number in the range of 1 to 40, preferably 3 to 30.
c- polyethylene glycol fatty acid esters of the following general structure
R₃ C(O) (OCH₂CH₂)ₙ OH
wherein R₃ is straight or branched, saturated or unsaturated alkyl group which may be synthetic or natural with a C chain length in the range of 7 to 39, preferably 9 to 29 and more preferably 9 to 23 and n is a number in the range of 5 to 40, preferably 9 to 30.
d- polypropylene glycol fatty acid esters of the following general structure
R₄ C(O) (OCH₂(CH₃)CH₂)ₙ OH
wherein R₄ is straight or branched, saturated or unsaturated alkyl group which may be synthetic or natural with a C chain length in the range of 7 to 39, preferably 9 to 29 and more preferably 9 to 23 and n is a number in the range of 1 to 40, preferably 9 to 30.
e- polyethylene glycol and polypropylene glycol ether of fatty alcohols of the following general structure
R₅ (OCH₂(CH₃)CH₂)ₙ₁ (OCH₂CH₂)ₙ₂ OH
wherein R₅ is straight or branched, saturated or unsaturated alkyl group which may be synthetic or natural with a C chain length in the range of 7 to 39, preferably 9 to 29 and more preferably 9 to 23 and n1 and n2 may be the same or different and are a number in the range of 1 to 40.
f- ethoxylated triglycerides.

8. The process according to any one of the claims 1 to 8 wherein the aqueous composition comprises one or more nonionic surfactant at a total concentration of 0.1 to 5%, preferably 0.2 to 4%, more preferably 0.25 to 3% and most preferably 0.3 to 2.5% by weight, calculated to the total composition.

9. The process according to one of the claims 1 to 9 wherein the aqueous composition comprises one or more aminated silicones according to general structure 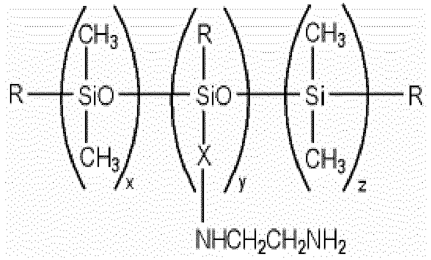 wherein R is the same or different OH or CH₃ or OCH₃ and X represents butyl, propyl, isopropyl or isobutyl preferably at a concentration in the range of 0.1 to 5% by weight calculated to the total composition.

10. The process according to one of the claims 1 to 10 wherein the aminated silicone is Bis(Hydroxy/Methoxy) Amodimethcone.

11. The process according to one of the claims 1 to 10 wherein the aqueous composition does not comprise quaternary ammonium surfactant at a concentration more than 10%, preferably 5% by weight of the total concentration of one or more cationic quaternary ammonium polymers having the cationic charge density defined in the preceding claims.

12. The process according to any of one of the proceeding claims wherein the aqueous composition comprises one or more of the following compounds
- Organic solvent,
- Fatty alcohol of the general structure
R₆-OH
wherein R₆ is straight or branched, saturated or unsaturated alkyl chain which may be synthetic or natural with a C chain length in the range of 12 to 24 which may as well be substituted with one or more groups on the alkyl chain,
- UV filter,
- Hair direct dye selected from cationic and neutral dyes,
- Natural plant extract,
- Protein hydrolyzate,
- Ubiquinone, and
- Nonionic polymer.
- Oil and/or oily substances selected from silicone oils, natural and synthetic oily, fatty alcohol dialkyl ethers and fatty acid fatty alcohol esters.

13. The process according to any one of the proceeding claims wherein the aqueous composition has a pH in the range of 3.0 to 5.5.

14. Use of the process according to any of the preceding claims for long lasting conditioning of the hair, especially improving combability and suppleness of human hair.

## Patentansprüche

1. Verfahren zum Behandeln von Haaren, wobei eine wässrige Zusammensetzung, welche ein oder mehrere kationische quartäre Ammoniumpolymere mit einer kationischen Ladungsdichte von 3,0 mÄq/g oder mehr aufweist und einen pH-Wert im Bereich von 3,0 bis 6,5 aufweist, auf die Haare aufgebracht wird, gegebenenfalls für eine Dauer von 1 min bis 15 min auf den Haaren belassen wird, gegebenenfalls aus den Haaren ausgespült wird und die Haartemperatur auf den Bereich von 80 °C bis 140 °C erhöht wird.

2. Verfahren nach Anspruch 1, wobei die Haare mit einem Haartrockner oder mit einer digitalen Haarerwärmungsvorrichtung erwärmt werden, mit einem Eisen, welches entweder flach sein kann oder bestimmte Oberflächenstrukturen aufweist, oder einem Eisen mit runder Form.

3. Verfahren nach Anspruch 1 oder 2, wobei das kationische quartäre Ammoniumpolymer eine Ladungsdichte von 3,5 mÄq/g bis 8 mÄq/g, mehr bevorzugt 4 mÄq/g bis 7,5 mÄq/g, am meisten bevorzugt 4,5 mÄq/g bis 7,0 mÄq/g und insbesondere 4,5 mÄq/g bis 6,5 mÄq/g aufweist.

4. Verfahren nach einem der Ansprüche 1 bis 3, wobei das kationische quartäre Ammoniumpolymer aus Polyquaternium-37, Polyquaternium-6, Polyquaternium-7 und Polyquaternium-16 ausgewählt ist, wobei es vorzugsweise Polyquaternium-37 ist.

5. Verfahren nach einem der Ansprüche 1 bis 5, wobei die wässrige Zusammensetzung ein oder mehrere kationische quartäre Ammoniumpolymere in einer Gesamtkonzentration von 0,1 Gewichts-% bis 5 Gewichts-% aufweist, bezogen auf die Gesamtzusammensetzung.

6. Verfahren nach einem der Ansprüche 1 bis 6, wobei die wässrige Zusammensetzung ein oder mehrere nichtionische Tenside aufweist.

7. Verfahren nach einem der Ansprüche 1 bis 7, wobei ein oder mehrere nichtionische Tenside ausgewählt sind aus
a- Fettalkoholethoxylaten der folgenden allgemeinen Struktur
R₁ (OCH₂CH₂)ₙ OH,
wobei R₁ für eine lineare oder verzweigte, gesättigte oder ungesättigte Alkylkette, welche synthetisch oder natürlich sein kann, mit einer C-Kettenlänge im Bereich von 8 bis 40, vorzugsweise 9 bis 30 und mehr bevorzugt 9 bis 24 steht und n eine Zahl im Bereich von 5 bis 40, vorzugsweise 9 bis 30 ist,
b- Polypropylenglykolether von Fettalkoholen der allgemeinen Struktur
R₂(OCH₂(CH₃)CH₂)ₙ OH,
wobei R₂ für einen linearen oder verzweigten, gesättigten oder ungesättigten Fettalkohol, welcher synthetisch oder natürlich sein kann, mit einer C-Kettenlänge im Bereich von 8 bis 40, vorzugsweise 9 bis 30 und mehr bevorzugt 9 bis 24 steht und n eine Zahl im Bereich von 1 bis 40, vorzugsweise 3 bis 30 ist,
c- Polyethylenglykol-Fettsäureestern der folgenden allgemeinen Struktur
R₃ C(O) (OCH₂CH₂)ₙ OH,
wobei R₃ für eine lineare oder verzweigte, gesättigte oder ungesättigte Alkylgruppe, welche synthetisch oder natürlich sein kann, mit einer C-Kettenlänge im Bereich von 7 bis 39, vorzugsweise 9 bis 29 und mehr bevorzugt 9 bis 23 steht und n eine Zahl im Bereich von 5 bis 40, vorzugsweise 9 bis 30 ist,
d- Polypropylenglykol-Fettsäureestern der folgenden allgemeinen Struktur
R₄ C(O)(OCH₂(CH₃)CH₂)ₙ OH,
wobei R₄ für eine lineare oder verzweigte, gesättigte oder ungesättigte Alkylgruppe, welche synthetisch oder natürlich sein kann, mit einer C-Kettenlänge im Bereich von 7 bis 39, vorzugsweise 9 bis 29 und mehr bevorzugt 9 bis 23 steht und n eine Zahl im Bereich von 1 bis 40, vorzugsweise 9 bis 30 ist,
e- Polyethylenglykol- und Polypropylenglykolether von Fettalkoholen der folgenden allgemeinen Struktur
R₅(OCH₂(CH₃)CH₂)ₙ₁ (OCH₂CH₂)ₙ₂ OH,
wobei R₅ für eine lineare oder verzweigte, gesättigte oder ungesättigte Alkylgruppe, welche synthetisch oder natürlich sein kann, mit einer C-Kettenlänge im Bereich von 7 bis 39, vorzugsweise 9 bis 29 und mehr bevorzugt 9 bis 23 steht und n₁ und n₂ gleich oder verschieden sind und eine Zahl im Bereich von 1 bis 40 sind.
f- ethoxylierten Triglyceriden.

8. Verfahren nach einem der Ansprüche 1 bis 8, wobei die wässrige Zusammensetzung ein oder mehrere nichtionische Tenside in einer Gesamtkonzentration von 0,1 Gewichts-% bis 5 Gewichts-%, vorzugsweise 0,2 Gewichts-% bis 4 Gewichts-%, mehr bevorzugt 0,25 Gewichts-% bis 3 Gewichts-% und am meisten bevorzugt 0,3 Gewichts-% bis 2,5 Gewichts-% aufweist, bezogen auf die Gesamtzusammensetzung.

9. Verfahren nach einem der Ansprüche 1 bis 9, wobei die wässrige Zusammensetzung ein oder mehrere aminierte Silikone der allgemeinen Struktur 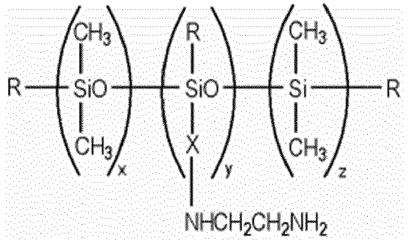 aufweist, wobei R gleich oder verschieden ist und für OH oder CH₃ oder OCH₃ steht und X für Butyl, Propyl, Isopropyl oder Isobutyl steht, vorzugsweise in einer Konzentration im Bereich von 0,1 Gewichts-% bis 5 Gewichts-%, bezogen auf die Gesamtzusammensetzung.

10. Verfahren nach einem der Ansprüche 1 bis 10, wobei das aminierte Silikon Bis(hydroxy/methoxy)amodimethicon ist.

11. Verfahren nach einem der Ansprüche 1 bis 10, wobei die wässrige Zusammensetzung quartäres Ammoniumtensid nicht in einer Konzentration von mehr als 10 Gewichts-%, vorzugsweise 5 Gewichts-%, der Gesamtkonzentration eines oder mehrerer kationischer quartärer Ammoniumpolymere aufweist, welche die Ladungsdichte aufweisen, die in den vorhergehenden Ansprüchen definiert ist.

12. Verfahren nach einem der vorhergehenden Ansprüche, wobei die wässrige Zusammensetzung eine oder mehrere der folgenden Verbindungen aufweist
- Organisches Lösungsmittel,
- Fettalkohol der allgemeinen Struktur
R₆-OH
wobei R₆ für eine lineare oder verzweigte, gesättigte oder ungesättigte Alkylkette, welche synthetisch oder natürlich sein kann, mit einer C-Kettenlänge im Bereich von 12 bis 24 steht, welche auch mit einer oder mehreren Gruppen an der Alkylkette substituiert sein kann,
- UV-Filter,
- Haar-Direktfarbstoff, ausgewählt aus kationischen und neutralen Farbstoffen,
- Natürlicher Pflanzenextrakt,
- Protein-Hydrolysat,
- Ubichinon, und
- Nichtionisches Polymer,
- Öl und/oder ölige Substanzen, ausgewählt aus Silikonölen, natürlichen und synthetischen öligen, Fettalkohol-Dialkylethern und Fettsäure-Fettalkoholestern.

13. Verfahren nach einem der vorhergehenden Ansprüche, wobei die wässrige Zusammensetzung einen pH-Wert im Bereich von 3,0 bis 5,5 aufweist.

14. Verwendung des Verfahrens nach einem der vorhergehenden Ansprüche zum dauerhaften Konditionieren der Haare, insbesondere Verbessern der Kämmbarkeit und Geschmeidigkeit menschlicher Haare.

## Revendications

1. Procédé de traitement des cheveux dans lequel une composition aqueuse comprenant un ou plusieurs polymères cationiques d'ammonium quaternaire ayant une densité de charge cationique de 3,0 mEq/g ou plus et ayant un pH dans la plage de 3,0 à 6,5 est appliquée sur les cheveux, est éventuellement laissée sur les cheveux pour une durée de 1 à 15 minutes, est éventuellement enlevée des cheveux par rinçage et la température des cheveux est élevée jusque dans la plage de 80 à 140 °C.

2. Procédé selon la revendication 1, dans lequel les cheveux sont chauffés avec un sèche-cheveux ou avec un appareil de chauffage des cheveux numérique, avec un fer qui peut être soit plat, soit avoir certaines structures de surface, ou un fer de forme ronde.

3. Procédé selon les revendications 1ou 2, dans lequel le polymère cationique d'ammonium quaternaire a une densité de charge de 3,5 à 8 mEq/g, plus préférentiellement de 4 à 7,5 mEq/g, idéalement de 4,5 à 7,0 mEq/g et en particulier de 4,5 à 6,5 mEq/g.

4. Procédé selon l'une quelconque des revendications 1 à 3, dans lequel le polymère cationique d'ammonium quaternaire est choisi parmi le Polyquaternium-37, le Polyquaternium-6, le Polyquaternium-7, et le Polyquaternium-16, c'est de préférence le Polyquaternium-37.

5. Procédé selon l'une quelconque des revendications 1 à 5, dans lequel la composition aqueuse comprend un ou plusieurs polymères cationiques d'ammonium quaternaire à une concentration totale de 0,1 à 5 % en poids, calculée par rapport à la concentration totale.

6. Procédé selon l'une quelconque des revendications 1 à 6, dans lequel la composition aqueuse comprend un ou plusieurs tensioactifs non ioniques.

7. Procédé selon l'une quelconque des revendications 1 à 7, dans lequel un ou plusieurs tensioactifs non ioniques sont choisis parmi
a- des éthoxylates d'alcools gras selon la structure générale suivante
R₁ (OCH₂CH₂)ₙ OH
où R₁ est une chaine alkyle linéaire ou ramifiée, saturée ou non saturée, qui peut être synthétique ou naturelle, avec une longueur de chaîne dans la plage de 8 à 40 C, de préférence de 9 à 30 et plus préférentiellement de 9 à 24 et n est un nombre dans la plage de 5 à 40, de préférence de 9 à 30.
b- des éthers d'alcools gras de polypropylène glycol selon la structure générale
R₂ (OCH₂(CH₃)CH₂)ₙ OH
où R₂ est un alcool gras linéaire ou ramifié, saturé ou non saturé, qui peut être synthétique ou naturel, avec une longueur de chaîne dans la plage de 8 à 40 C, de préférence de 9 à 30 et plus préférentiellement de 9 à 24, et n est un nombre dans la plage de 1 à 40, de préférence de 3 à 30.
c- des esters d'acides gras de polyéthylène glycol selon la structure générale suivante
R₃ C(O) (OCH₂CH₂)ₙ OH
où R₃ est une chaine alkyle linéaire ou ramifiée, saturée ou non saturée, qui peut être synthétique ou naturelle avec une longueur de chaîne dans la plage de 7 à 39 C, de préférence de 9 à 29, et plus préférentiellement de 9 à 23 et n est un nombre dans la plage de 5 à 40, de préférence de 9 à 30.
d- des esters d'acides gras de polypropylène glycol selon la structure générale suivante
R₄ C(O)(OCH₂(CH₃)CH₂)ₙ OH
où R₄ est une chaine alkyle linéaire ou ramifiée, saturée ou non saturée, qui peut être synthétique ou naturelle, avec une longueur de chaîne dans la plage de 7 à 39 C, de préférence de 9 à 29, et plus préférentiellement de 9 à 23 et n est un nombre dans la plage de 1 à 40, de préférence de 9 à 30.
e- des éthers d'alcools gras de polyéthylène glycol et de polypropylène glycol selon la structure générale suivante
R₅ (OCH₂(CH₃)CH₂)ₙ₁ (OCH₂CH₂)ₙ₂ OH
où R₅ est une chaine alkyle linéaire ou ramifiée, saturée ou non saturée, qui peut être synthétique ou naturelle, avec une longueur de chaîne dans la plage de 7 à 39 C, de préférence de 9 à 29, et plus préférentiellement de 9 à 23, et n1 et n2 peuvent être identiques ou différents et sont des nombres dans la plage de 1 à 40.
f- des triglycérides éthoxylés.

8. Procédé selon l'une quelconque des revendications 1 à 8, dans lequel la composition aqueuse comprend un ou plusieurs tensioactifs non ioniques à une concentration totale de 0,1 à 5 %, de préférence de 0,2 à 4 %, plus préférentiellement de 0,25 à 3 %, et idéalement de 0,3 à 2,5 % en poids, calculée par rapport à la concentration totale.

9. Procédé selon l'une des revendications 1 à 9, dans lequel la composition aqueuse comprend un ou plusieurs silicones aminés selon la structure générale 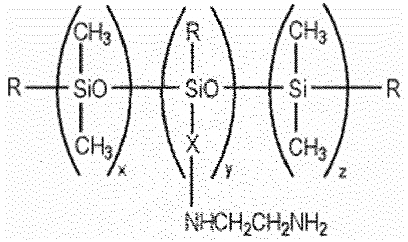 où les R sont identiques ou différents et sont des groupes OH, ou CH₃, ou OCH₃, et X représente un groupe butyle, propyle, isopropyle ou isobutyle, de préférence à une concentration dans la plage de 0,1 à 5 % en poids, calculée par rapport à la concentration totale.

10. Procédé selon l'une des revendications 1 à 10, dans lequel le silicone aminé est la bis(hydroxy/méthoxy) amodiméthicone.

11. Procédé selon l'une des revendications 1 à 10, dans lequel la composition aqueuse ne comprend pas de tensioactif d'ammonium quaternaire à une concentration supérieure à 10 %, de préférence 5 % en poids de la concentration totale d'un ou de plusieurs polymères cationiques d'ammonium quaternaire ayant la densité de charge cationique définie dans les revendications précédentes.

12. Procédé selon l'une quelconque des revendications précédentes, dans lequel la composition aqueuse comprend un ou plusieurs parmi les composés suivants
- un solvant organique,
- un alcool gras selon la structure générale
R₆-OH
où R₆ est une chaine alkyle linéaire ou ramifiée, saturée ou non saturée, qui peut être synthétique ou naturelle, avec une longueur de chaîne dans la plage de 12 à 24 C qui peut être substituée soit par un, soit par plusieurs groupes sur la chaîne alkyle,
- un filtre UV,
- un colorant pour cheveux direct choisi parmi des colorants cationiques et neutres,
- un extrait de plante naturel,
- un produit d'hydrolyse de protéine,
- de l'ubiquinone, et
- un polymère non ionique,
- de l'huile et/ou des substances huileuses choisies parmi des huiles silicones, des éthers dialkyle d'acides gras huileux naturels et synthétiques et des ester d'alcools d'acides gras.

13. Procédé selon l'une quelconque des revendications précédentes, dans lequel la composition aqueuse a un pH dans la plage de 3,0 à 5,5.

14. Utilisation du procédé selon l'une quelconque des revendications précédentes pour un conditionnement de longue durée des cheveux, améliorant en particulier le coiffage et la souplesse des cheveux humains.
